# EUROPEAN PATENT APPLICATION

(11) **EP 4 721 811 A1**
(43) Date of publication of application: **08.04.2026**
(21) Application number: 24204939.3
(22) Date of filing: 07.10.2024
(51) Int. Cl.: A61N 1/39, A61B 5/361

(54) **DISPLAYING SHOCKABLE CARDIAC RHYTHMS IN MANUAL DEFIBRILLATION MODE**

(71) Applicant: Koninklijke Philips N.V., 5656 AG Eindhoven (NL)
(72) Inventor: JORGENSON, Dawn Blilie, Eindhoven (NL); LIU, Chenguang, Eindhoven (NL)
(74) Representative: Philips Intellectual Property & Standards

(57) **Abstract**

A monitor/defibrillator (10) employing a defibrillator controller (30) for, in a manual defibrillation mode, analyzing an unfiltered ECG data including cardiopulmonary resuscitation artifacts for detecting any presence of an unfiltered shockable cardiac rhythm within the unfiltered ECG data, and analyzing a filtered ECG data excluding cardiopulmonary resuscitation artifacts for detecting any presence of a filtered shockable cardiac rhythm within the filtered ECG data. The monitor/defibrillator (10) further employs a monitor controller (40) for, in the manual defibrillation mode, displaying an unfiltered ECG waveform and a filtered ECG waveform, and for delineating a color coding of a shock advised segment of the display of the filtered ECG waveform as an indication of a detection by the defibrillator controller (30) of a presence of the unfiltered shockable cardiac rhythm within the unfiltered ECG data and/or of a detection by the defibrillator controller (30) of a presence of the filtered shockable cardiac rhythm within the filtered ECG data.

## Description

### FIELD OF THE INVENTION

The present disclosure generally relates to an operation by a manual defibrillator in a manual defibrillation mode. The present disclosure particularly relates to managing a display, by a manual defibrillator operating in a manual defibrillation mode, of shockable cardiac rhythm(s) within an electrocardiogram (ECG) waveform.

### BACKGROUND OF THE INVENTION

When a manual defibrillator (e.g., Philips Intrepid) is utilized in a manual defibrillation mode during a rescue attempt of a patient, a clinician may perform cardiopulmonary resuscitation (CPR), then pause the chest compressions of the CPR to observe cardiac rhythm(s) of an electrocardiogram (ECG) waveform displayed on the monitor screen. From such observation of the cardiac rhythm(s), the clinician will decide whether a shock should be given to the patient based on his/her knowledge and experience related to an interpretation of the cardiac rhythm(s) of the ECG waveform. Typically, the ECG waveform during CPR chest compressions is too obscure to tell if a cardiac rhythm of the ECG waveform is shockable, and this is why a CPR pause is needed for the clinician to assess the cardiac rhythm(s) of the ECG waveform.

As such, the manual defibrillator industry is also striving to facilitate accurate and timely interpretations of cardiac rhythm(s) of an ECG waveform by a clinician when the manual defibrillator is operating in the manual defibrillation mode, particularly during CPR chest compressions.

### SUMMARY OF THE INVENTION

The present disclosure utilizes electrocardiogram (ECG) filtering techniques and shockable cardiac rhythm detection algorithms to display comprehensive visual information to a clinician operating a manual defibrillator in a manual defibrillation mode during a cardiopulmonary resuscitation (CPR) of a patient in a manner that facilitates accurate and timely interpretation(s) of cardiac rhythm(s) of an ECG waveform by the clinician as a basis for the clinician's decision to shock or not shock the patient. More particularly, the comprehensive visual information is provided to alert the clinician of a detected shockable cardiac rhythm within the ECG waveform primarily during an administration of chest compressions by the clinician to the patient, and additionally during a cessation of chest compressions by the clinician to the patient.

For purposes of describing and claiming the present disclosure,
(1) terms of the art of the present disclosure, but not limited to, "manual defibrillation", "defibrillator", "ECG monitor", "electrocardiogram", "CPR", "waveform" and "cardiac rhythm" are to be interpreted as known in the art of the present disclosure and as exemplary described in the present disclosure; and
(2) the phrase "manual defibrillator" broadly encompasses any device/system employing an integration or a segregation of an ECG monitor configured in accordance with the present disclosure and a defibrillator configured in accordance with the present disclosure to implement a manual defibrillation mode as would be appreciated by those having ordinary skill in the art of the present disclosure.

The present disclosure may be embodied as (1) a manual defibrillator operable in a manual defibrillation mode, (2) a defibrillator controller operable in a manual defibrillation mode, (3) a monitor controller operable in a manual defibrillation mode, and (4) a manual defibrillation method.

Various embodiments of a manual defibrillator of the present disclosure encompass a defibrillator controller and a monitor controller.

In operation, the defibrillator controller is configured, in a manual defibrillation mode, to (1) analyze unfiltered electrocardiogram (ECG) data including cardiopulmonary resuscitation artifacts for detecting any presence of an unfiltered shockable cardiac rhythm within the unfiltered ECG waveform, and (2) analyze filtered ECG data excluding cardiopulmonary resuscitation artifacts for detecting any presence of a filtered shockable cardiac rhythm within the filtered ECG waveform.

Further in operation, the monitor controller is configured, in the manual defibrillation mode, to (1) display an unfiltered ECG waveform and a filtered ECG waveform, and (2) delineate a color coding of a shock advised segment of the display of the filtered ECG waveform as an indication of (a) a detection by the defibrillator controller of a presence of the unfiltered shockable cardiac rhythm within the unfiltered ECG data and/or (b) a detection by the defibrillator controller of a presence of the filtered shockable cardiac rhythm within the filtered ECG data.

Various embodiments of a defibrillator controller of the present disclosure encompass a a non-transitory machine-readable storage medium encoded with instructions for execution by one or more processors, in a manual defibrillation mode, to (1) analyze unfiltered electrocardiogram (ECG) data including cardiopulmonary resuscitation artifacts for detecting any presence of an unfiltered shockable cardiac rhythm within the unfiltered ECG data, and (2) analyze a filtered ECG data excluding cardiopulmonary resuscitation artifacts for detecting any presence of a filtered shockable cardiac rhythm within the filtered ECG data.

Various embodiments of a monitor controller of the present disclosure encompass a a non-transitory machine-readable storage medium encoded with instructions for execution by one or more processors, in a manual defibrillation mode, to (1) display unfiltered ECG waveform and filtered ECG waveform, and (2) delineate a color coding of a shock advised segment of the display of the filtered ECG waveform as an indication of (a) a detection by the defibrillator controller of a presence of the unfiltered shockable cardiac rhythm within the unfiltered ECG data and/or (b) a detection by the defibrillator controller of a presence of the filtered shockable cardiac rhythm within the filtered ECG data.

Various exemplary embodiments of a manual defibrillation method in accordance with the present disclosure are executable by a manual defibrillator operating in a manual defibrillation mode.

The embodiments of manual defibrillation method of the present disclosure encompass the manual defibrillator (1) analyzing an unfiltered ECG data including cardiopulmonary resuscitation artifacts for detecting any presence of an unfiltered shockable cardiac rhythm within the unfiltered ECG data, (2) analyzing the filtered ECG data excluding cardiopulmonary resuscitation artifacts for detecting any presence of a filtered shockable cardiac rhythm within the filtered ECG data, (3) displaying a unfiltered ECG waveform and a filtered ECG waveform; and (4) delineating a color coding of a shock advised segment of the display of the filtered ECG waveform as an indication of at least one of a detection by the manual defibrillator of a presence of the unfiltered shockable cardiac rhythm within the unfiltered ECG data and a detection by the manual defibrillator of a presence of the filtered shockable cardiac rhythm within the filtered ECG data.

The foregoing exemplary embodiments and other embodiments of the present disclosure as well as various structures and advantages of the present disclosure will become further apparent to those having ordinary skill in the art from the following detailed description of various embodiments of the present disclosure read in conjunction with the accompanying drawings. The detailed description and drawings are merely illustrative of the present disclosure rather than limiting, the scope of the present disclosure being defined by the appended claims and equivalents thereof.

### BRIEF DESCRIPTION OF THE DRAWINGS

The present disclosure will present in detail the following description of exemplary embodiments with reference to the following Figures wherein:
FIG. 1 illustrates an exemplary embodiment of a manual defibrillator in accordance with the present disclosure;
FIG. 2 illustrates an exemplary embodiment of a monitor display screen in accordance with the present disclosure;
FIG. 3 illustrates exemplary embodiments of a defibrillator controller and a monitor controller in accordance with the present disclosure;
FIG. 4 illustrates an exemplary operation of the defibrillator controller of FIG. 3 in accordance with the present disclosure;
FIGS. 5 and 6 illustrate an exemplary operation of the monitor controller of FIG. 3 in accordance with the present disclosure;
FIGS. 7A-7F illustrate exemplary monitor display screens derived from the exemplary operation of the defibrillator controller shown in FIG. 4 and the exemplary operation of the monitor controller shown in FIGS. 5 and 6.
FIG. 8 illustrates an exemplary embodiment of a controller in accordance with the present disclosure.

### DETAILED DESCRIPTION OF THE PREFERRED EMBODIMENTS

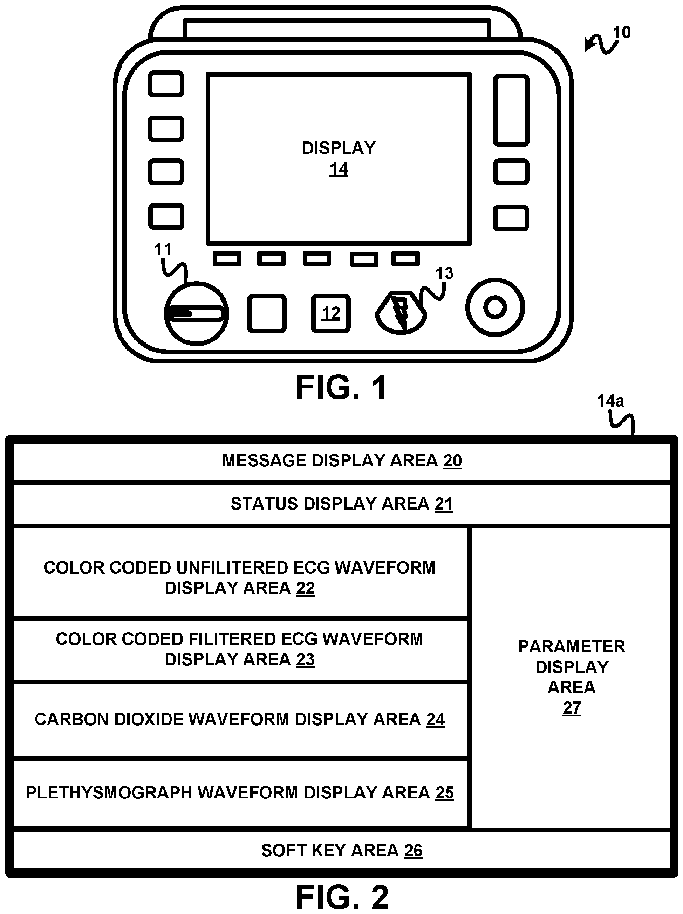

To facilitate an understanding of the present disclosure, the following description of FIGS. 1-8 describes and teaches exemplary embodiments of a manual defibrillator, a defibrillator controller, a monitor controller, and a manual defibrillation method in accordance with the present disclosure. From the description of FIGS. 1-8, those having ordinary skill in the art of the present disclosure will appreciate how to apply the present disclosure to make and use additional embodiments of a manual defibrillator, a defibrillator controller, a monitor controller, and a manual defibrillation method of the present disclosure.

Referring to FIG. 1, an exemplary manual defibrillator 10 of the present disclosure that is utilized to shock a patient experiencing cardiac arrest in an attempt to restore a heart of the patient to a normal cardiac rhythm as would be appreciated by those having ordinary skill in the art. As shown, manual defibrillator 10 incorporates a settings dial 11 for enabling a clinician to set various parameters of the shock (e.g., an energy level of the shock), a charge control 12, a shock control 13 and a display 14.

In accordance with the present disclosure, an exemplary monitor display screen 14a includes a message display area 20, a status display area 21, a carbon dioxide waveform display area 24, a plethysmograph waveform display area 25, a soft key area 26 and a parameter display area 27 as known in the art of the present disclosure.

Exemplary monitor display screen 14a further includes a color coded unfiltered ECG waveform display area 23 and a color coded filtered ECG waveform display area 24 in accordance with the present disclosure for display comprehensive visual information to a clinician operating manual defibrillator 10 during a cardiopulmonary resuscitation (CPR) of a patient in a manner that facilitates accurate and timely interpretation(s) of cardiac rhythm(s) of an ECG waveform by the clinician as a basis for the clinician's decision to shock or not shock the patient.

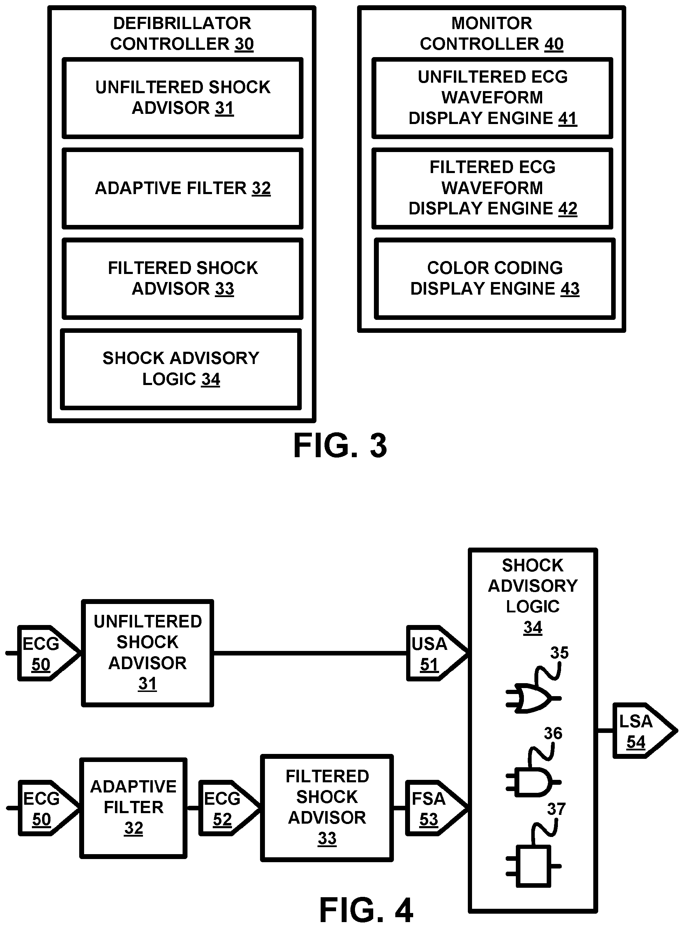

To this end, referring to FIG. 3, an exemplary defibrillator controller 30 of the present disclosure employs an unfiltered ECG shock advisor 31, an adaptive filter 32, a filtered ECG shock advisor 33 and a shock advisory lock 34.

As known in the art of the present disclosure, unfiltered shock advisor 31 has the capability to detect any shockable cardiac rhythms within an unfiltered ECG waveform including CPR artifacts during an administration of chest compressions to the heart of the patient, adaptive filter 32 has the capability to remove an CPR artifacts within an ECG waveform, and filtered shock advisor 33 has the capability to detect any shockable cardiac rhythm(s) within a filtered ECG waveform excluding CPR artifacts during an administration of chest compressions to the heart of the patient.

In accordance with the present disclosure, shock advisory logic 34 has the capability to perform a logical operation on the ECG waveform outputs of unfiltered shock advisor 31 and filtered shock advisor 33 to provide a logical shock advisory 54 as a waveform or an enable/disable signal.

For example, FIG. 4 illustrates unfiltered shock advisor 31 outputting an unfiltered shock advisory 51 based on an analysis of an unfiltered ECG waveform 50 directed to detect any shockable cardiac rhythm(s) within an unfiltered ECG waveform including CPR artifacts during an administration of chest compressions to the heart of the patient.

Still referring to FIG. 4, adaptive filter 32 is shown filtering the CPR artifacts from unfiltered ECG waveform 50 to derive a filtered ECG waveform 52 excluding the CPR artifacts, and filtered shock advisor 33 is shown outputting a filtered shock advisory 53 based on an analysis of a filtered ECG waveform 52 directed to detect any shockable cardiac rhythm(s) within an unfiltered ECG waveform excluding CPR artifacts during the administration of chest compressions to the heart of the patient.

Unfiltered shock advisory 51 and filtered shock advisory 53 are binary signals, each indicating shock advice (e.g., a "0") or no-shock advice (e.g., a "1").

In one exemplary embodiment of shock advisory logic 34, an OR gate 35 is utilized to derive logical shock advisory 54 indicating shock advice (e.g., a "1") when unfiltered shock advisory 51 is indicating shock advice (e.g., a "1") and/or filtered shock advisory 53 is indicating shock advice (e.g., a "1").

OR gate 35 is further utilized to derive logical shock advisory 54 indicating no-shock advice (e.g., a "0") when unfiltered shock advisory 51 is indicating no-shock advice (e.g., a "0")and filtered shock advisory 53 is indicating no-shock advice (e.g., a "0").

In another exemplary embodiment of shock advisory logic 34, an AND gate 36 is utilized to derive logical shock advisory 54 indicating shock advice (e.g., a "1") when unfiltered shock advisory 51 is indicating shock advice (e.g., a "1") and filtered shock advisory 53 is indicating shock advice (e.g., a "1").

AND gate 36 is further utilized to derive logical shock advisory 54 indicating no-shock advice (e.g., a "0") when unfiltered shock advisory 51 is indicating no-shock advice (e.g., a "0") and/or filtered shock advisory 53 is indicating no-shock advice (e.g., a "0").

In yet another exemplary embodiment, of shock advisory logic 34, a logical unit 37 is utilized to derive logical shock advisory 54 indicating shock advice (e.g., a "11") when unfiltered shock advisory 51 is indicating shock advice (e.g., a "1") and filtered shock advisory 53 is indicating shock advice (e.g., a "1").

Logical unit 37 is further utilized to derive logical shock advisory 54 indicating no-shock advice (e.g., a "00") when unfiltered shock advisory 51 is indicating no-shock advice (e.g., a "0") and filtered shock advisory 53 is indicating no-shock advice (e.g., a "0").

Logical unit 37 is further utilized to derive logical shock advisory 54 indicating indecisive shock advice (e.g., a "01" or "10") when unfiltered shock advisory 51 is indicating no-shock advice (e.g., a "0") and filtered shock advisory 53 is indicating shock advice (e.g., a "1"), or when unfiltered shock advisory 51 is indicating shock advice (e.g., a "1") and filtered shock advisory 53 is indicating no-shock advice (e.g., a "0").

Referring back to FIG. 3, an exemplary monitor controller 40 of the present disclosure employs an unfiltered ECG waveform display engine 41, a filtered ECG waveform display engine 42 and a color-coded display engine 43.

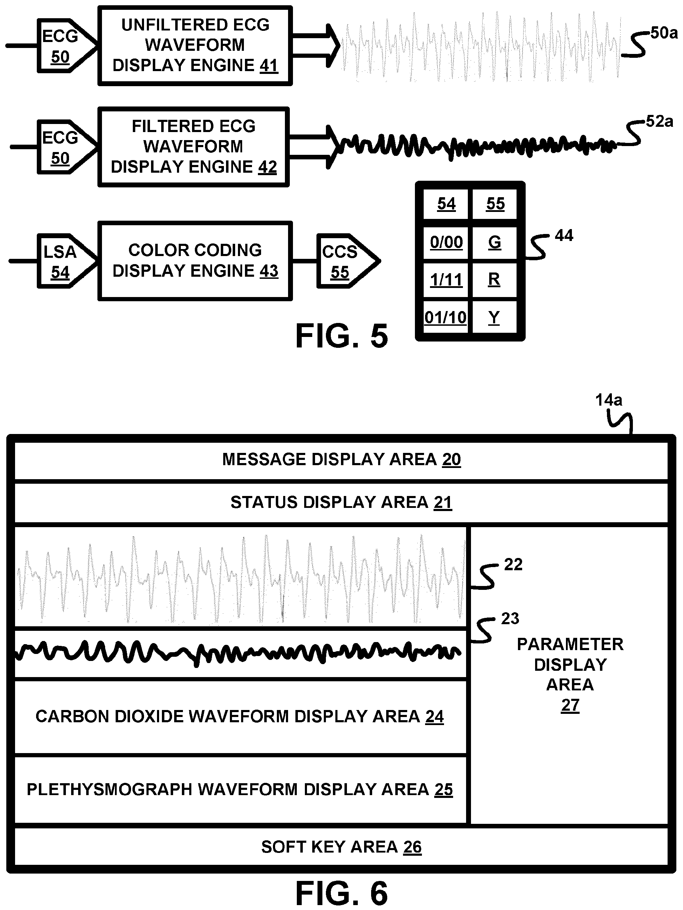

As known in the art of the present disclosure, unfiltered ECG waveform display engine 41 displays unfiltered ECG waveform 50a within display area 22 of screen 14a (FIG. 2) as exemplary shown in FIGS. 5 and 6, and filtered ECG waveform display engine 42 displays filtered ECG waveform 52a within display area 23 of screen 14a (FIG. 2) as exemplary shown in FIGS. 5 and 6.

Referring back to FIG. 3, in accordance with the present disclosure, color-coded display engine 43 color-codes segments of unfiltered ECG waveform 50 and/or filtered ECG waveform 52 to provide visual information of any detection of shockable cardiac rhythm(s) within waveforms 50 and/or 52. To this end, color-coded display engine 43 outputs color codes 55 that are synchronized with the ECG waveforms whereby segments of the ECG waveforms may be delineating with the color codes 55.

In an exemplary embodiment of color-coded display engine 43, an exemplary truth table 44 is utilized whereby a segment of an ECG waveform will be color-coded green (G) color if that segment corresponds to logical shock advisory 54 indicating no-shock advice (e.g., a "0" or a "00"), a segment of an ECG waveform will be color-coded red (R) color if that segment corresponds to logical shock advisory 54 indicating shock advice (e.g., a "1" or a "11"), and a segment of an ECG waveform will be color-coded yellow (Y) color if that segment corresponds to logical shock advisory 54 indicating indecisive shock advice (e.g., a "01" or a "10").

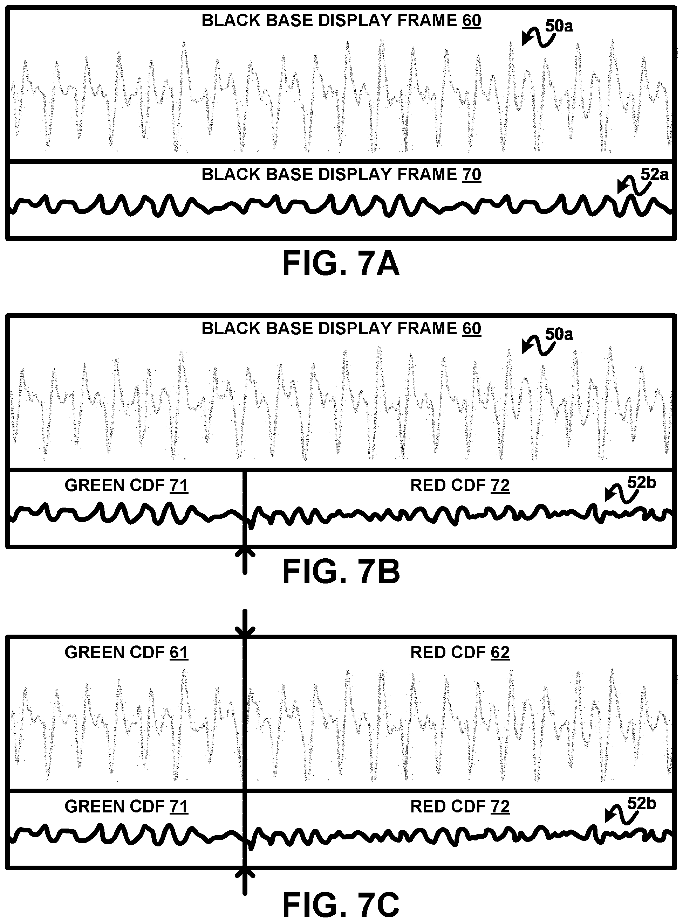

FIGS. 7A-7C illustrate exemplary color-codings of the ECG waveforms.

Specifically, FIG. 7A illustrates unfiltered ECG waveform 50a and filtered ECG waveform 52a within basic black image frames 60 and 70, respectively, prior to a commencement of the color-coding feature of the present invention.

FIG. 7B illustrates a no-shock segment of filtered ECG waveform 52a delineated within a green coded image frame 71 and a shock segment of filtered ECG waveform 52a delineated within a red coded image frame 72.

FIG. 7C illustrates a no-shock segment of unfiltered ECG waveform 50a delineated within a green coded image frame 61 and a shock segment of unfiltered ECG delineated waveform 50a within a red coded image frame 62.

FIG. 7C further illustrates a no-shock segment of filtered ECG waveform 52a delineated within a green coded image frame 71 and a shock segment of filtered ECG waveform 52a delineated within a red coded image frame 72.

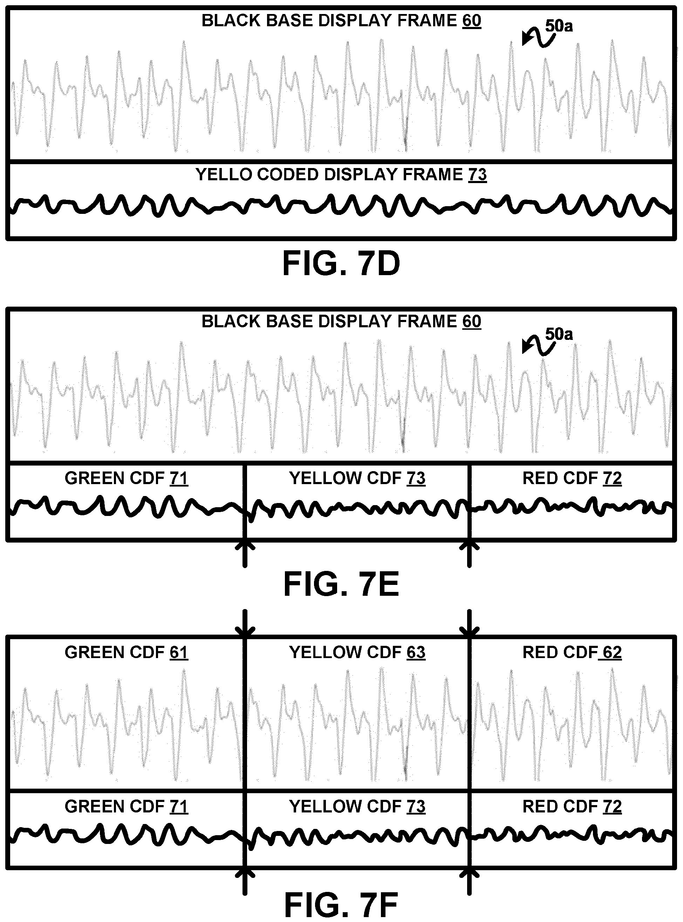

FIG. 7D illustrates unfiltered ECG waveform 50a is shown within a basic black image frame 60 and filtered ECG waveform 52a is shown within yellow color coded image frame 73 prior to a commencement of the color-coding feature of the present invention.

FIG. 7E illustrates a no-shock segment of filtered ECG waveform 52a delineated within a green coded image frame 71, an indecisive a shock segment of filtered ECG waveform 52a delineated within a yellow coded image frame 73, and a shock segment of filtered ECG waveform 52a delineated within a red coded image frame 72.

FIG. 7F illustrates a no-shock segment of unfiltered ECG waveform 50a delineated within a green coded image frame 61, an indecisive segment of unfiltered ECG waveform 50a delineated within a yellow coded image frame 63, and a shock segment of unfiltered ECG delineated waveform 50a within a red coded image frame 62.

FIG. 7F illustrates a no-shock segment of filtered ECG waveform 52a delineated within a green coded image frame 71, an indecisive a shock segment of filtered ECG waveform 52a delineated within a yellow coded image frame 73, and a shock segment of filtered ECG delineated waveform 52a within a red coded image frame 72.

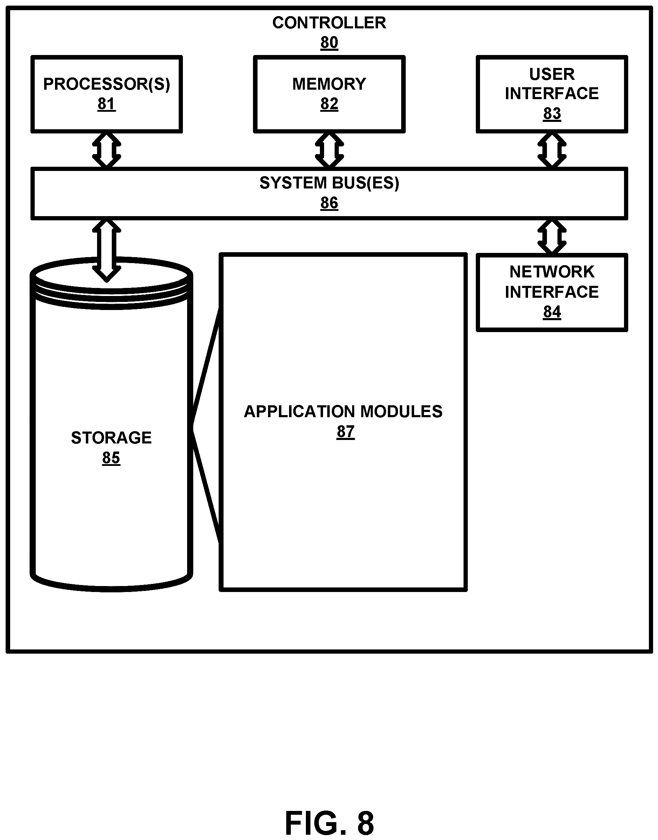

Referring to FIG. 8, shown is an exemplary embodiment of controller 80 that includes one or more processor(s) 81, memory 82, a user interface 83, a network interface 84, and a storage 85 interconnected via one or more system bus(es) 86.

Each processor 81 can be any hardware device, as known in the art of the present disclosure or hereinafter conceived, capable of executing instructions stored in memory 82 or storage or otherwise processing data. In a non-limiting example, the processor(s) 81 can include a microprocessor, field programmable gate array (FPGA), application-specific integrated circuit (ASIC), or other similar devices.

The memory 82 can include various memories, as known in the art of the present disclosure or hereinafter conceived, including, but not limited to, L1, L2, or L3 cache or system memory. In a non-limiting example, the memory 82 can include static random access memory (SRAM), dynamic RAM (DRAM), flash memory, read only memory (ROM), or other similar memory devices.

The user interface 83 can include one or more devices, as known in the art of the present disclosure or hereinafter conceived, for enabling communication with a user such as an administrator. In a non-limiting example, the user interface can include a command line interface or graphical user interface that can be presented to a remote terminal via the network interface 84.

The network interface 84 can include one or more devices, as known in the art of the present disclosure or hereinafter conceived, for enabling communication other components of a medical device. In a non-limiting example, the network interface 84 can include a network interface card (NIC) configured to communicate according to the Ethernet protocol. Additionally, the network interface 84 may implement a TCP/IP stack for communication according to the TCP/IP protocols. Various alternative or additional hardware or configurations for the network interface 84 will be apparent.

The storage 85 can include one or more machine-readable storage media, as known in the art of the present disclosure or hereinafter conceived, including, but not limited to, read-only memory (ROM), random-access memory (RAM), magnetic disk storage media, optical storage media, flash-memory devices, or similar storage media. In various non-limiting embodiments, the storage 85 can store instructions for execution by the processor(s) 81 or data upon with the processor(s) 81 may operate. For example, the storage 85 may store a base operating system for controlling various basic operations of the hardware.

The storage 85 can also store an application module(s) 87 in the form of executable software/firmware for implementing the present disclosure as previously described in the present disclosure.

In an exemplary embodiment of controller 80 as a defibrillator controller of the present disclosure, the application module(s) 87 include the unfiltered shock advisor 31, the adaptive filter 32, the filtered shock advisor 33, and the shock advisory logic 34 as previously described herein in connection with FIG. 3 of the present disclosure. In an exemplary embodiment of controller 80 as a monitor controller of the present disclosure, the application module(s) 87 include the unfiltered ECG waveform display engine 41, the filtered ECG waveform display engine 42 and the color coded display engine 43 as previously described herein in connection with FIG. 3 of the present disclosure.

In an exemplary embodiment of controller 80 being an integration of a defibrillator controller and a monitor controller of the present disclosure, the application module(s) 87 include the unfiltered shock advisor 31, the adaptive filter 32, the filtered shock advisor 33, the shock advisory logic 34 the unfiltered ECG waveform display engine 41, the filtered ECG waveform display engine 42 and the color coded display engine 43 as previously described herein in connection with FIG. 3 of the present disclosure.

From the description of FIGS. 1-8 herein, those having ordinary skill in the art will appreciate the numerous benefits of the present disclosure including, but not limited to, a display of comprehensive visual information to a clinician operating a manual defibrillator in a manual defibrillation mode during a cardiopulmonary resuscitation (CPR) of a patient in a manner that facilitates accurate and timely interpretation(s) of cardiac rhythm(s) of an ECG waveform by the clinician as a basis for the clinician's decision to shock or not shock the patient.

The present disclosure has been described with reference to the preferred embodiments. Modifications and alterations may occur to others upon reading and understanding the preceding detailed description. It is intended that the invention be construed as including all such modifications and alterations insofar as they come within the scope of the appended claims or the equivalents thereof.

Further, as one having ordinary skill in the art shall appreciate in view of the teachings provided herein, features, elements, components, etc. disclosed and described in the present disclosure/specification and/or depicted in the appended Figures and/or recited in the Claims can be implemented in various combinations of hardware and software, and provide functions which may be combined in a single element or multiple elements. For example, the functions of the various features, elements, components, etc. shown/illustrated/depicted in the Figures and/or recited in the Claims can be provided through the use of dedicated hardware as well as hardware capable of executing software in association with appropriate software. When provided by a processor, the functions can be provided by a single dedicated processor, by a single shared processor, or by a plurality of individual processors, some of which can be shared and/or multiplexed. Moreover, explicit use of the term "processor" or "controller" should not be construed to refer exclusively to hardware capable of executing software, and can implicitly include, without limitation, digital signal processor ("DSP") hardware, memory (e.g., read only memory ("ROM") for storing software, random access memory ("RAM"), non-volatile storage, etc.) and virtually any means and/or machine (including hardware, software, firmware, combinations thereof, etc.) which is capable of (and/or configurable) to perform and/or control a process.

Moreover, all statements herein reciting principles, aspects, and exemplary embodiments of the present disclosure, as well as specific examples thereof, are intended to encompass both structural and functional equivalents thereof. Additionally, it is intended that such equivalents include both currently known equivalents as well as equivalents developed in the future (e.g., any elements developed that can perform the same or substantially similar functionality, regardless of structure). Thus, for example, it will be appreciated by one having ordinary skill in the art in view of the teachings provided herein that any block diagrams presented herein can represent conceptual views of illustrative system components and/or circuitry embodying the principles of the invention. Similarly, one having ordinary skill in the art should appreciate in view of the teachings provided herein that any flow charts, flow diagrams and the like can represent various processes which can be substantially represented in computer readable storage media and so executed by a computer, processor or other device with processing capabilities, whether or not such computer or processor is explicitly shown.

Having described preferred and exemplary embodiments of the present disclosure, which embodiments are intended to be illustrative and not limiting, it is noted that modifications and variations can be made by persons having ordinary skill in the art in view of the teachings provided herein, including the appended Figures and claims. It is therefore to be understood that changes can be made in/to the preferred and exemplary embodiments of the present disclosure which are within the scope of the present disclosure and exemplary embodiments disclosed, described and taught herein.

Moreover, it is contemplated that corresponding and/or related systems incorporating and/or implementing the device or such as may be used/implemented in a device in accordance with the present disclosure are also contemplated and considered to be within the scope of the present disclosure. Further, corresponding and/or related method for manufacturing and/or using a device and/or system in accordance with the present disclosure are also contemplated and considered to be within the scope of the present disclosure.

## Claims

1. A monitor/defibrillator (10), comprising:
a defibrillator controller (30) configured, in a manual defibrillation mode, to:
analyze an unfiltered electrocardiogram (ECG) data including cardiopulmonary resuscitation artifacts for detecting any presence of an unfiltered shockable cardiac rhythm within the unfiltered ECG waveform;
analyze a filtered ECG data excluding cardiopulmonary resuscitation artifacts for detecting any presence of a filtered shockable cardiac rhythm within the filtered ECG data; and
a monitor controller (40) configured, in the manual defibrillation mode, to:
display an unfiltered ECG waveform and a filtered ECG waveform; and
delineate a color coding of a shock advised segment of the display of the filtered ECG waveform as an indication of at least one of a detection by the defibrillator controller (30) of a presence of the unfiltered shockable cardiac rhythm within the unfiltered ECG data and of a detection by the defibrillator controller (30) of a presence of the filtered shockable cardiac rhythm within the filtered ECG data.

2. The monitor/defibrillator (10) of claim 1, wherein the monitor controller (40) is further configured to:
delineating a color coding of a no-shock advised segment of the display of the filtered ECG waveform as an indication of both a non-detection by the defibrillator controller (30) of the presence of the unfiltered shockable cardiac rhythm within the unfiltered ECG data and a non-detection by the defibrillator controller (30) of the presence of the filtered shockable cardiac rhythm within the filtered ECG data.

3. The monitor/defibrillator (10) of claim 1, wherein the monitor controller (40) is further configured to:
delineating a color coding of a shock advised segment of the display of the unfiltered ECG waveform as an indication of the at least one of the detection by the defibrillator controller (30) of the presence of the unfiltered shockable cardiac rhythm within the unfiltered ECG data and the detection by the defibrillator controller (30) of the presence of the filtered shockable cardiac rhythm within the filtered ECG data.

4. The monitor/defibrillator (10) of claim 1, wherein the monitor controller (40) is further configured to:
delineating a color coding of a no-shock advised segment of the display of the unfiltered ECG waveform as an indication of both a non-detection by the defibrillator controller (30) of the presence of the unfiltered shockable cardiac rhythm within the unfiltered ECG data and a non-detection by the defibrillator controller (30) of the presence of the filtered shockable cardiac rhythm within the filtered ECG data.

5. The monitor/defibrillator (10) of claim 1, wherein the defibrillator controller (30) includes:
an unfiltered shock advisor (31) configured to generate an unfiltered shock advisory (31) derived from an analysis of the unfiltered ECG data for detecting any presence of the unfiltered shockable cardiac rhythm within the unfiltered ECG data; and
a filtered shock advisor (33) configured to generate a filtered shock advisory (33) derived from an analysis of the filtered ECG data for detecting any presence of the filtered shockable cardiac rhythm within the unfiltered ECG data.

6. The monitor/defibrillator (10) of claim 5, wherein the defibrillator controller (30) further includes:
an adaptive filter (32) configured to derive the filtered ECG data excluding cardiopulmonary resuscitation artifacts by filtering out the cardiopulmonary resuscitation artifacts from the unfiltered ECG data.

7. The monitor/defibrillator (10) of claim 5,
wherein the defibrillator controller (30) further includes a shock advisory logic (34) configured to derive a logical shock advisory by performing a logical operation on the unfiltered shock advisory (31) and the filtered shock advisory (33); and
wherein the logical shock advisory waveform is indicative of at least one of any detected presence of the unfiltered shockable cardiac rhythm within the unfiltered ECG data and of any detected presence of the filtered shockable cardiac rhythm within the filtered ECG data.

8. The monitor/defibrillator (10) of claim 7, wherein the monitor controller (40) further includes:
a color coding display engine (43) configured to delineate a color coding of the shock advised segment of the display of the filtered ECG waveform responsive to the logical shock advisory waveform indicating at least one of a detection by the unfiltered shock advisor (31) of the presence of the unfiltered shockable cardiac rhythm within the unfiltered ECG data and of a detection by the filtered shock advisor (33) of the presence of the filtered shockable cardiac rhythm within the filtered ECG data.

9. The monitor/defibrillator (10) of claim 7, wherein the monitor controller (40) includes:
a color coding display engine (43) configured to delineate a color coding of a no-shock advised segment of the display of the filtered ECG waveform responsive to the logical shock advisory waveform indicating both a non-detection by the unfiltered shock advisor (31) of the presence of the unfiltered shockable cardiac rhythm within the unfiltered ECG data and a non-detection by the filtered shock advisor (33) of the presence of the filtered shockable cardiac rhythm within the filtered ECG data.

10. The monitor/defibrillator (10) of claim 7, wherein the monitor controller (40) includes:
a color coding display engine (43) configured to delineate a color coding of a shock advised segment of the display of the unfiltered ECG waveform responsive to the logical shock advisory waveform indicating at least one of a detection by the unfiltered shock advisor (31) of the presence of the unfiltered shockable cardiac rhythm within the unfiltered ECG data and of a detection by the filtered shock advisor (33) presence of the filtered shockable cardiac rhythm within the filtered ECG data.

11. The monitor/defibrillator (10) of claim 7, wherein the monitor controller (40) includes:
a color coding display engine (43) configured to delineate a color coding of a no-shock advised segment of the display of the unfiltered ECG waveform responsive to the logical shock advisory waveform indicating both a non-detection by the unfiltered shock advisor (31) of the presence of the unfiltered shockable cardiac rhythm within the unfiltered ECG data and a non-detection a detection by the filtered shock advisor (33) of the presence of the filtered shockable cardiac rhythm within the filtered ECG data.

12. A manual defibrillation method executable by a monitor/defibrillator (10) operating in a manual defibrillation mode, the manual defibrillation method comprising:
analyzing, by the monitor/defibrillator (10), an unfiltered ECG data including cardiopulmonary resuscitation artifacts for detecting any presence of an unfiltered shockable cardiac rhythm within the unfiltered ECG data;
analyzing, by the monitor/defibrillator (10), the filtered ECG data excluding cardiopulmonary resuscitation artifacts for detecting any presence of a filtered shockable cardiac rhythm within the filtered ECG data;
displaying, by the monitor/defibrillator (10), an unfiltered ECG waveform and a filtered ECG waveform; and
delineating a color coding, by the monitor/defibrillator (10), of a shock advised segment of the display of the filtered ECG waveform as an indication of at least one of a detection by the monitor/defibrillator (10) of a presence of the unfiltered shockable cardiac rhythm within the unfiltered ECG data and a detection by the monitor/defibrillator (10) of a presence of the filtered shockable cardiac rhythm within the filtered ECG data.

13. The manual defibrillation method of claim 12, further comprising:
delineating a color coding, by the monitor/defibrillator (10), of a no-shock advised segment of the display of the filtered ECG waveform as an indication of both a non-detection by the defibrillation/monitor of the presence of the unfiltered shockable cardiac rhythm within the unfiltered ECG data and a non-detection by the monitor/defibrillator (10) of the presence of the filtered shockable cardiac rhythm within the filtered ECG data.

14. The manual defibrillation method of claim 12, further comprising:
delineating a color coding, by the monitor/defibrillator (10), of a shock advised segment of the display of the unfiltered ECG waveform as an indication of the at least one of the detection by the monitor/defibrillator (10) of the presence of the unfiltered shockable cardiac rhythm within the unfiltered ECG data and the detection by the monitor/defibrillator (10) of the presence of the filtered shockable cardiac rhythm within the filtered ECG data.

15. The manual defibrillation method of claim 12, further comprising:
delineating a color coding, by the monitor/defibrillator (10), of a no-shock advised segment of the display of the unfiltered ECG waveform as an indication of both a non-detection by the monitor/defibrillator (10) of the presence of the unfiltered shockable cardiac rhythm within the unfiltered ECG data and a non-detection by the monitor/defibrillator (10) of the presence of the filtered shockable cardiac rhythm within the filtered ECG data.
